# FASCICULE DE BREVET EUROPEEN

(11) **EP 2 721 408 B1**
(45) Date de publication et mention de la délivrance du brevet: **25.10.2017**
(21) Numéro de dépôt: 12730871.6
(22) Date de dépôt: 18.06.2012
(51) Int. Cl.: G01N 33/50, G01N 33/569

(54) **MÉTHODE DE DOSAGE IN VITRO PAR TECHNIQUE IMMUNOLOGIQUE**
IN-VITRO-TESTVERFAHREN MITTELS IMMUNOLOGISCHER TECHNIK
IN VITRO ASSAY METHOD USING IMMUNOLOGICAL TECHNIQUE

(30) Priorité: 20.06.2011 FR 1155415
(43) Date de publication de la demande: 23.04.2014
(73) Titulaire: BASF Beauty Care Solutions France SAS, 69007 Lyon (FR)
(72) Inventeur: BONNET, Sébastien, F-01800 Saint Jean De Niost (FR); RIVAL, Delphine, F-69360 Ternay (FR)
(74) Mandataire: Marro, Nicolas
(86) Numéro de dépôt international: PCT/EP2012/061603
(87) Numéro de publication internationale: WO 2012/175454

(56) Documents cités:
- EP-A2- 0 387 777
- WO-A2-03/062830
- GB-A- 2 438 999
- US-A1- 2006 079 439
- US-A1- 2009 104 201
- DIQUELOU ARMELLE ET AL: "Relationship between endothelial tissue factor and thrombogenesis under blood flow conditions", THROMBOSIS AND HAEMOSTASIS, SCHATTAUER GMBH, DE; US, vol. 74, no. 2, 1 janvier 1995 (1995-01-01), pages 778-783, XP009153780, ISSN: 0340-6245 cité dans la demande
- RYAN J ET AL: "TUMOR NECROSIS FACTOR-INDUCED ENDOTHELIAL TISSUE FACTOR IS ASSOCIATED WITH SUBENDOTHELIAL MATRIX VESICLES BUT IS NOT EXPRESSED ON THE APICAL SURFACE", BLOOD, vol. 80, no. 4, 1992, pages 966-974, XP002663035, ISSN: 0006-4971 cité dans la demande
- DOOLEY AUDREY ET AL: "Modulation of collagen type I, fibronectin and dermal fibroblast function and activity, in systemic sclerosis by the antioxidant epigallocatechin-3-gallate", RHEUMATOLOGY (OXFORD), vol. 49, no. 11, novembre 2010 (2010-11), pages 2024-2036, XP002663036, ISSN: 1462-0324
- DOOLEY ET AL: "Effect of nitric oxide and peroxynitrite on type I collagen synthesis in normal and scleroderma dermal fibroblasts", FREE RADICAL BIOLOGY AND MEDICINE, ELSEVIER SCIENCE, US, vol. 43, no. 2, 27 juin 2007 (2007-06-27), pages 253-264, XP022132572, ISSN: 0891-5849, DOI: 10.1016/J.FREERADBIOMED.2007.04.017

## Description

La présente invention concerne une nouvelle méthode de dosage in vitro des molécules de la matrice extracellulaire synthétisées par des cellules en culture et ses utilisations sous forme de kit de mesure in vitro et/ou de méthode de screening d'ingrédients cosmétiques et/ou pharmaceutiques.

La matrice extracellulaire (MEC) tient un rôle essentiel dans la structure des tissus du corps humain et animal, en particulier par ses fonctions de soutien, d'adhérence et de régulation des échanges cellulaires. La MEC est constituée en grande partie de glycoprotéines, protéines et glycosaminoglycannes. Ces molécules sont synthétisées sous forme native dans les cellules en contact avec la MEC. Du compartiment intracellulaire, elles sont excrétées en dehors des cellules. Après des phénomènes de maturation, elles s'organisent et s'agencent en un réseau qui forme la MEC. Elles sont alors sous leur forme fonctionnelle. Les molécules de la MEC sont particulièrement étudiées dans les domaines cosmétiques et pharmaceutiques où de nombreux ingrédients visent à stimuler leur synthèse et ainsi améliorer l'état général du tissu concerné.

In vitro, les cellules en culture dans des conditions appropriées produisent leur matrice extracellullaire et constituent un modèle d'études particulièrement simple, notamment pour mesurer l'activité de synthèse par les cellules d'une ou plusieurs molécules constituant la MEC et l'influence d'ingrédients sur cette activité. Dans ce type de modèle de cellules en culture, les molécules de la MEC sont présentes, dans différents compartiments de culture et éventuellement sous différentes formes notamment de précurseurs: intracellulaire, dans le milieu de culture, ou contenues dans la MEC.

Les techniques de mesures classiques consistent à mesurer la quantité de molécules de la MEC dans le milieu de culture. Ainsi, selon la technique la plus courante, on dose dans le milieu de culture par technique ELISA des molécules de la MEC telles que les collagènes, éventuellement sous leur forme précurseur telles que le procollagène. Une autre technique plus couteuse, plus contraignante et plus globale, consiste à inclure dans le milieu de culture des molécules radiomarquées utilisés par les cellules pour la synthèse des molécules de la MEC et à mesurer globalement l'intensité de la radioactivité ainsi obtenue dans la totalité des compartiments. Par exemple, la proline tritiée est utilisée pour mesurer la quantité de collagène globalement produit par les cellules en culture. Ces techniques permettent de rendre compte de l'activité de synthèse des cellules en culture mais pas de la fonctionnalité des molécules ainsi synthétisées c'est-à-dire de la quantité de molécules dans la MEC.

Seule l'analyse immunohistochimique permet de déterminer et seulement en partie la fonctionnalité des molécules de la MEC ainsi synthétisées. Cette technique consiste à éliminer le milieu de culture, marquer avec des anticorps dirigés contre les molécules de la MEC étudiées, puis lire et/ou visualiser la fluorescence. Cette technique permet de quantifier les molécules contenues dans le compartiment intracellulaire, extracellulaire et dans la MEC et surtout de visualiser leur localisation. Cette technique est néanmoins lourde à mettre en oeuvre et nécessite des équipements couteux. La technique en elle-même ne permet pas en outre d'analyser un grand nombre d'ingrédients. Par ailleurs, elle est subjective et dépend de l'expérimentateur. Ainsi, avant la présente invention aucune mesure quantitative objective ne permettait de déterminer la quantité de molécules fonctionnelles c'est-à-dire les molécules réellement intégrées dans la MEC et en particulier les molécules qui constituent la MEC de manière suffisamment rapide, fiable, simple et prédictive. Par ailleurs, aucune méthode ne permettait le screening d'ingrédients d'intérêt cosmétiques et/ou pharmaceutiques.

Or, une synthèse importante de molécules de la MEC par des cellules en culture mesurée dans le milieu intracellulaire et/ou extracellulaire ne se traduit pas nécessairement par une quantité plus importante de molécules fonctionnelles c'est-à-dire de molécules dans la MEC.

En effet, la Demanderesse a pu constater que certains ingrédients actifs cosmétiques permettent de stimuler globalement la synthèse de collagène de la MEC sans toutefois visualiser d'amélioration de la fonctionnalité du collagène c'est-à-dire pas d'augmentation de la teneur du collagène dans la MEC. La Demanderesse a également constaté que certains ingrédients améliorent la fonctionnalité du collagène c'est-à-dire sa teneur dans la MEC sans toutefois mesurer d'augmentation de la synthèse de procollagène. (exemples 6 et 7)

Les techniques de l'art antérieur ne sont donc pas appropriées pour quantifier la fonctionnalité des molécules de la MEC c'est à dire leur teneur dans la MEC en ce qu'elles ne sont pas directes ni assez fiables, prédictives, reproductibles, répétables et spécifiques. Par ailleurs, elles ne permettent pas le screening ou « criblage » d'ingrédients cosmétiques et/ou pharmaceutiques susceptibles d'améliorer la fonctionnalité des molécules de la MEC d'une manière satisfaisante et d'en visualiser les effets dans la MEC.

La demande de brevet GB2438999 concerne une méthode pour analyser l'expression de LOXL dans des cultures de cellules de muscle lisse. Cette demande décrit notamment un test comprenant les étapes de culture de cellule de muscle lisse, de collecte du milieu de culture, de lyse des cellules avec un tampon de lyse, de collecte du lysat cellulaire, et de dosage des LOX et LOXL par une méthode immunologique.

La demande de brevet WO03/062830 décrit une méthode de criblage de la fibronectine ayant des domaines EDIIIA+ et EDIIIB+. Cette demande décrit notamment l'utilisation de NH₄OH pour la lyse des cellules.

Les articles Diquelou A. et al, 1995, Relationship between endothelial tissue factor and thrombogenesis under blood conditions,Thrombosis and haemostasis, vol 74, n°2, p778-783 et Ryan J et al, 1992, Tumor necrosis factor-induced endothelial tissue factor is associated with subendothelial matrix vesicles but is not expressed on the apical surface, Blood, vol 80, n°4, p966-974 ont décrit le dosage de TF synthétisé par des HUVECS après 4 ou 8h de culture. Néanmoins, de telles durées de culture sont insuffisantes pour que des cellules en culture synthétisent une MEC et pour étudier la fonctionnalité des molécules constitutives et/ou contenues dans la MEC. Ces expériences ont d'ailleurs une finalité différente.

Il existait donc un besoin de disposer d'une technique de dosage de molécules de la MEC synthétisées par des cellules en culture qui permette de doser directement, quantitativement, de manière fiable, simple, répétable, prédictive et spécifique la fonctionnalité des molécules de la MEC c'est à dire leur teneur dans la MEC. Il existait par ailleurs un besoin de disposer d'une technique de dosage non dénaturante de la MEC et qui permette de quantifier et visualiser la molécule étudiée au sein de la MEC et de l'étudier in situ, notamment dans sa forme fonctionnelle et dans ses interactions avec les autres molécules de la MEC.

La Demanderesse vient maintenant de découvrir de manière surprenante et inattendue que ce problème peut être résolu en effectuant une étape de lyse spécifique des cellules en culture et un dosage direct sur la MEC des molécules d'intérêt. La méthode selon l'invention fournit ainsi une solution technique au problème de l'art antérieur et présente l'avantage d'être rapide, miniaturisable, et de permettre le screening d'un grand nombre d'ingrédients pour leur capacité à augmenter ou diminuer la quantité de molécules dans la MEC, avantageusement sous forme automatisable. La méthode selon l'invention a également l'avantage de ne pas détruire la MEC et de permettre à la fois la quantification et la visualisation des molécules de la MEC.

La présente invention a pour objet une méthode de dosage *in vitro* par technique immunologique d'au moins une molécule de la matrice extracellulaire (MEC) synthétisée par des cellules en culture comprenant au moins les étapes :
a) une étape de mise en culture des cellules à confluence pendant au moins 24 heures,
b) une étape préférentielle de prélèvement du milieu de culture,
c) une étape de lyse des cellules par une solution d'amine quaternaire,
d) une étape de prélèvement du lysat cellulaire et de dosage d'ADN et/ou de ladite molécule de la MEC dans le lysat cellulaire,
e) une étape de dosage par technique immunologique de ladite molécule de la MEC dans la matrice extracellulaire,
et dans laquelle les cellules en culture sont maintenues en culture au stade de confluence pendant une durée qui va de 24 à 96 heures avant la réalisation de l'étape c).

Cette méthode permet avantageusement de doser la molécule de la MEC dans les différents compartiments : intracellulaire, dans le milieu de culture et dans la MEC.

La présente description concerne une méthode de dosage in vitro par technique immunologique d'au moins une molécule de la matrice extracellulaire synthétisée par des cellules en culture comprenant au moins les étapes :
a) une étape de mise en culture des cellules et préférentiellement culture des cellules à confluence pendant au moins 24 heures,
b) une étape préférentielle de prélèvement du milieu de culture,
c) une étape de lyse des cellules par une solution d'amine quaternaire, préférentiellement par une solution d'amine quaternaire à une concentration comprise entre 100µM et 2M,
d) une étape de prélèvement du lysat cellulaire,.
e) une étape de dosage par technique immunologique de ladite molécule dans la matrice extracellulaire.

La présente description concerne en outre une méthode de dosage in vitro par technique immunologique d'au moins une molécule de la matrice extracellulaire synthétisée par des cellules en culture comprenant au moins les étapes :
a) une étape de mise en culture des cellules et préférentiellement culture des cellules à confluence pendant au moins 24 heures,
b) une étape de prélèvement du milieu de culture et de dosage de la molécule de la MEC et/ou un précurseur de la molécule de la MEC,
c) une étape de lyse des cellules par une solution d'amine quaternaire, préférentiellement par une solution d'amine quaternaire à une concentration comprise entre 100µM et 2M,
d) une étape de prélèvement du lysat cellulaire et de dosage de l'ADN et/ou de dosage de la molécule de la MEC et/ou un précurseur de la molécule de la MEC,
e) une étape de dosage par technique immunologique de ladite molécule de la MEC contenue dans la matrice extracellulaire.

La présente invention a également pour objet l'utilisation de la méthode de dosage selon l'invention pour le screening et/ou l'étude in vitro d'au moins un ingrédient d'intérêt cosmétique et/ou pharmaceutique pour ses propriétés à augmenter ou diminuer la quantité d'une molécule dans la matrice extracellulaire.

La présente invention a également pour objet une méthode de screening et/ou d'étude *in vitro* d'au moins un ingrédient d'intérêt cosmétique et/ou pharmaceutique pour ses propriétés à augmenter ou diminuer la quantité d'une molécule dans la matrice extracellulaire comprenant au moins les étapes :
a) une étape de mise en culture des cellules en présence de l'ingrédient,
b) préférentiellement une étape de prélèvement du milieu de culture et encore préférentiellement de dosage de la molécule de la MEC et/ou un précurseur de la molécule de la MEC dans ledit milieu de culture ainsi prélevé,
c) une étape de lyse des cellules par une solution d'amine quaternaire,
d) une étape de prélèvement du lysat cellulaire et de dosage de l'ADN et/ou de dosage de la molécule de la MEC et/ou un précurseur de la molécule de la MEC,
e) une étape de dosage par technique immunologique de ladite molécule de la MEC contenue dans la matrice extracellulaire,
et dans laquelle la culture de cellules dans le milieu de culture contenant l'ingrédient cosmétique et/ou pharmaceutique est effectuée pendant au moins 24 heures.

La présente description concerne également une méthode de screening et/ou d'étude in vitro d'au moins un ingrédient d'intérêt cosmétique et/ou pharmaceutique pour ses propriétés à augmenter ou diminuer la quantité d'une molécule dans la matrice extracellulaire comprenant au moins les étapes :
a) une étape de mise en culture des cellules et culture des cellules en présence de l'ingrédient, préférentiellement pendant au moins 24 heures après confluence,
b) préférentiellement une étape de prélèvement du milieu de culture et encore préférentiellement de dosage de la molécule de la MEC et/ou un précurseur de la molécule de la MEC dans ledit milieu de culture ainsi prélevé,
c) une étape de lyse des cellules par une solution d'amine quaternaire, préférentiellement par une solution d'amine quaternaire à une concentration comprise entre 100µM et 2M
d) une étape de prélèvement du lysat cellulaire et préférentiellement de dosage de l'ADN et/ou de dosage de la molécule de la MEC et/ou un précurseur de la molécule de la MEC,
e) une étape de dosage par technique immunologique de ladite molécule de la MEC contenue dans la matrice extracellulaire.

Un autre objet de la présente invention est l'utilisation d'un kit de dosage par méthode immunologique dans une méthodes de dosage ou de screening selon l'invention, ledit kit comprenant une solution d'amine quaternaire, préférentiellement une solution d'amine quaternaire à une concentration comprise entre 100µM et 2M, et un anticorps dirigé contre la molécule de la MEC, éventuellement couplé à un anticorps secondaire:
Un autre objet de la présente invention est un procédé de sélection d'une solution de lyse utile dans la méthode de dosage ou dans la méthode de screening selon l'invention caractérisé en ce que la méthode de dosage selon l'invention est mise en oeuvre avec une solution de lyse à tester pendant au moins 10 min, préférentiellement 10 min, et le ratio du résultat du dosage de la molécule de la MEC obtenus à l'étape e) sur le résultat du dosage de l'ADN dans le lysat cellulaire à l'étape d) est comparé à celui obtenu avec une solution de lyse d'hydroxyde d'ammonium à 20mM appliquée pendant le même temps, préférentiellement 10 minutes.

Selon l'invention, on entend par « molécules de la MEC », les molécules organiques qui constituent et/ou sont contenues dans la matrice extracellulaire et qui sont synthétisées par des cellules, d'origine humaines ou animales.

Il s'agit notamment des protéines constitutives de la MEC, en particulier sélectionnées parmi :
- La famille des collagènes de la MEC: les collagènes fibrillaires notamment de type I, III, V, les collagènes FACITS (Fibril Associated Collagen with Interupted Triple Helix) notamment de type VI, XII, XIV, XVI, les collagènes IV et VII, les collagènes XXII, XXVII, XVIII
- La famille des fibres élastiques de la MEC : l'élastine, la tropoélastine, les protéines associées à l'élastine notamment la fibrilline 1, les lysyl oxidases, notamment LOX et LOXL, l'EBP (Elastine Binding Protein), les fibullines 3 et 5, les Emilin 1 et 2.

Il s'agit notamment des protéoglycanes constitutifs de la MEC, en particulier les protéoglycanes sécrétés choisis parmi le Perlecan, le versican, les protéoglycanes riches en leucine (SLRPs) notamment la decorine, le biglycan et le lumican.

Il s'agit également des glycoprotéines constitutifs de la MEC, notamment la fibronectine, la laminine, la SPARC (Secreted Protein Rich in Cystein), la tenascine, le nidogène 1.

Il s'agit en outre des glycosaminoglycans (GAG) constitutifs de la MEC, en particulier l'acide hyaluronique, heparane sulfate, dermatan sulfate, chondroitine sulfate.

Il s'agit également des facteurs de croissance de la MEC qui sont des protéines contenues dans la MEC, notamment le VEGF, le PDGF, le HGF, les FGF, et en particulier FGF2 et FGF 7.

La présente invention permet très avantageusement de doser les molécules constitutives de la MEC et de les visualiser in situ dans le puit c'est-à-dire sans destruction de la MEC.

Certaines molécules de la MEC existent dans d'autres compartiments que la MEC sous forme de précurseur.

Les « molécules de la MEC » désignent les molécules dans la MEC ou dans d'autres compartiments notamment intracellulaire et extracellulaire dans le milieu de culture, éventuellement sous forme de précurseurs.

Selon l'invention, on entend par « précurseur de molécule de la MEC » une forme native et/ou intermédiaire de la molécule de la MEC, synthétisée par la cellule, avant de constituer ou être contenue dans la MEC. Ces formes subissent des phénomènes de maturation avant d'être dans la MEC et sont ainsi souvent désignées par les préfixes « Pro-» ou «Tropo- ». Il s'agit par exemple les procollagènes, les tropocollagènes, la proélastine, la tropoélastine.

Selon l'invention, on entend par « cellule(s) en culture », les cellules humaines ou animales capables de synthétiser une ou plusieurs molécules de la MEC. Ces cellules en culture sont cultivées selon la méthode selon la présente invention et la ou les molécules de la MEC qu'elles ont synthétisées sont dosées selon la présente invention. Elles sont préférentiellement de type différencié, notamment sous forme de précurseurs cellulaires différentiés ou cellules différentiés non précurseurs. Elles peuvent être normales, mutées ou immortalisées, cultivées en monocouche éventuellement en coculture avec un ou plusieurs autres types cellulaires capables ou non de synthétiser une ou plusieurs molécules de la MEC. Ces cellules sont notamment des cellules stromales, préférentiellement fibroblastes, ostéoblastes et/ou adipoblastes, préadipocytes, adipocytes, des cellules épithéliales, préférentiellement kératinocytes ou des cellules endothéliales. Elles sont extraites de biopsies, préférentiellement de peau, ou en lignées cellulaires. Selon un mode de réalisation préférentiel, la présente invention est tout particulièrement avantageuse pour l'étude des molécules constitutives de la MEC synthétisées par des fibroblastes en culture. En effet, une MEC fonctionnelle est essentielle dans la structure des tissus cutanés et la méthode selon la présente invention rend cet accès possible dans le puit de culture, y compris après 2 à 4 jours de culture de fibroblastes postconfluence.

Selon l'invention, on entend par « méthode immunologique », les techniques de dosage quantitatif par anticorps dirigé contre des protéines ou des sucres.

Il s'agit des techniques de dosage enzyme-substrat de type EIA (Enzyme Immuno Assay), notamment ELISA (Enzyme-Linked Immunosorbent Assay), de radiolabelling dit RIA (radioimmunoassay) et de mesure de fluorescence dit FIA (Fluorescente immunoassay) en particulier en fluorescence en temps retardé dite TRF (Time Resolved fluorescence).

Selon l'invention, on entend par « ingrédient d'intérêt cosmétique et/ou pharmaceutique », une ou plusieurs molécules naturelles et/ou de synthèses, et/ou un extrait végétal, éventuellement synthétisé et/ou modifié par génie biologique, en particulier par fermentation par microorganismes, dont les propriétés sur les molécules de la MEC sont évaluées pour ses capacités à augmenter ou diminuer la quantité de molécule dans la MEC.

Selon un aspect, l'invention est une méthode de dosage in vitro par technique immunologique d'au moins une molécule de la matrice extracellulaire synthétisée par des cellules en culture selon la revendication 1 comprenant au moins les étapes successives :
- étape de mise en culture des cellules
- une étape préférentielle de prélèvement du milieu de culture
- étape de lyse des cellules par une solution d'amine quaternaire
- étape de prélèvement du lysat cellulaire et de dosage d'ADN et/ou de ladite molécule de la MEC dans le lysat cellulaire
- étape de dosage par technique immunologique de ladite molécule de la MEC dans la matrice extracellulaire.

La méthode selon l'invention permet de doser au moins une molécule de la MEC et avantageusement une ou deux, encore préférentiellement au moins deux.

Selon un mode préférentiel, les molécules de la MEC sont choisies parmi les protéines constitutives de la MEC, les glycoprotéines constitutives de la MEC, les glycoaminoglycanes constitutives de la MEC, les protéoglycanes constitutives de la MEC et les facteurs de croissance contenues dans la MEC. Encore préférentiellement, elles sont choisies parmi les collagènes de type I, III, V, VI, XII, XIV, XVI, IV et VII, l'élastine, la tropoélastine, la fibrilline 1, LOX, LOXL, l'EBP (Elastine Binding Protein), les fibullines 3 et 5, les Emilin 1 et 2, le Perlecan, le versican, la decorine, le biglycan, le lumican, la fibronectine, la laminine, l'acide hyaluronique, heparane sulfate, le VEGF, le PDGF, le FGF-2, FGF-7 , et HGF.

Selon un mode particulièrement avantageux, les molécules de la MEC sont choisies parmi les collagènes de type I, III, V, XVIII, IV, VII, le perlecan, la fibronectine, le FGF-2, l'élastine, l'acide hyaluronique. Selon un mode préféré, les molécules de la MEC sont choisies parmi les collagènes de type I, III, V, XVIII, IV et VII.

Les cellules capables de synthétiser les molécules de la MEC sont mises en culture in vitro préférentiellement en monocouche selon les techniques habituelles en la matière. Elles sont ensemencées sur support de culture approprié, préférentiellement une plaque de culture cellulaire comprenant des puits, et cultivées dans un milieu de culture approprié jusqu'au stade de confluence, préférentiellement entre 80 et 100% de confluence, encore préférentiellement entre 90 et 100%, encore préférentiellement 100%.

Pour l'utilisation de la méthode en screening moyen débit, les plaques sont avantageusement des plaques de 96 ou 384 puits. De manière classique les plaques peuvent être « coatées » c'est-à-dire traitées pour améliorer l'adhérence des cellules par un revêtement qui peut être notamment des molécules constitutives de la MEC. Dans manière préférentielle, les plaques seront non coatées ou coatées avec une molécule différente de celle dont la mesure est effectuée et/ou dont on étudie la teneur dans la MEC. Selon un mode préférentiel, les cellules en culture sont choisies parmi des fibroblastes, préférentiellement dermiques et/ou des kératinocytes et/ou adipocytes, avantageusement humaines, et préférentiellement extraites de biopsies, préférentiellement de peau, préférentiellement des cellules humaines normales c'est-à-dire non mutées non immortalisées. Les études effectuées ont en effet montré que la méthode selon l'invention convient particulièrement bien à ces types cellulaires.

C'est généralement au stade de confluence que les cellules vont commencer à sécréter les molécules de la MEC. Ainsi pour la méthode de dosage selon l'invention, les cellules sont maintenues en culture au stade de confluence pendant une durée allant de 24 à 96 heures, préférentiellement pendant 48 heures. Ainsi pour la méthode de screening selon l'invention, les cellules sont maintenues en culture au stade de confluence pendant une durée d'au moins 24 heures et préférentiellement jusqu'à 6 jours, préférentiellement allant de 24 à 96 heures, et encore préférentiellement pendant 48 heures. Selon un mode particulièrement avantageux des méthodes de l'invention, le collagène est la molécule de la MEC dosée et la culture de fibroblastes est avantageusement maintenue pendant environ 48 heures.

Selon un mode de réalisation, à confluence, un ingrédient d'intérêt cosmétique et/ou pharmaceutique est ajouté dans le milieu de culture. Il peut également s'agir d'un ingrédient témoin de stimulation ou inhibition de molécules de la MEC. Préférentiellement, le témoin de stimulation est choisi parmi préférentiellement la vitamine C et le TGF beta, et avantageusement lorsque les cellules en culture sont des fibroblastes. La vitamine C est alors avantageusement utilisée à une dose comprise dans une gamme allant de 5µM à 100µM, encore préférentiellement à 50µM. Le TGF beta est alors avantageusement utilisé à une dose allant de 1ng/ml à 100ng/ml, préférentiellement à 10ng/ml.

De manière préférentielle pour la méthode de dosage selon l'invention, la culture de cellules à confluence dans le milieu de culture contenant l'ingrédient d'intérêt cosmétique et/ou pharmaceutique est effectuée pendant une durée allant de 24 à 96 heures, préférentiellement pendant 48 heures.

De manière préférentielle pour la méthode de screening selon l'invention, la culture de cellules à confluence dans le milieu de culture contenant l'ingrédient d'intérêt cosmétique et/ou pharmaceutique est effectuée pendant au moins 24 heures, préférentiellement jusqu'à 6 jours et préférentiellement de 24 à 96 heures, préférentiellement pendant 48 heures.

Une étape de prélèvement du milieu de culture totale ou partielle est alors préférentiellement effectuée. Selon un mode avantageux, la molécule de la MEC étudiée et contenue dans le milieu de culture, éventuellement sous une forme précurseur, est dosée par méthode immunologique, préférentiellement par dosage type ELISA.

La méthode selon l'invention comprend alors une étape de lyse des cellules en culture avec une solution d'amine quaternaire, préférentiellement une solution aqueuse, encore préférentiellement une solution d'ammonium éventuellement sous forme de sels. Selon un mode préférentiel, la solution d'amine quaternaire est choisie parmi une solution de chlorure d'ammonium, une solution d'hydroxyde d'ammonium et leurs mélanges.

Selon l'invention, on entend par « solution d'amine quaternaire » une solution dans laquelle la forme quaternaire de l'amine représente au moins 51% des formes de l'amine.

Selon un mode préférentiel, la solution d'amine quaternaire contient seulement l'amine quaternaire en solution aqueuse. Selon un autre mode préférentiel, la solution d'amine quaternaire à un pH basique, préférentiellement compris entre 10 et 13, encore préférentiellement de 11.

Les résultats présentés dans l'exemple 3 mettent en évidence les avantages des solutions d'amine quaternaire au regard des solutions classiques de lyse cellulaire. De manière surprenante, la solution d'amine quaternaire présente en effet les meilleurs taux de lyse des cellules et de dosage des molécules de la MEC.

Lors de cette étape de lyse, la solution d'amine quaternaire est mise en contact avec les cellules en culture, dans un état de confluence et préférentiellement après retrait total ou partiel du milieu de culture.

La solution d'amine quaternaire est appliquée à une concentration suffisante pour lyser les cellules. Les études menées par la Demanderesse ont mise en évidence qu'une concentration suffisante pour lyser les cellules est généralement comprise dans une gamme allant de 100 µM à 2M. Selon un mode préférentiel, la concentration de la solution d'amine quaternaire est de 1 mM à 200mM, encore préférentiellement de 10mM à 100mM encore préférentiellement 20mM. Dans le cas particulier ou le milieu de culture n'a pas été prélevé ou seulement partiellement, le facteur de dilution est considéré pour ajuster la concentration de la solution d'amine quaternaire.

Ces études effectuées dont une partie est présentée dans les exemples 2 montrent notamment que le temps de lyse préférentiel est compris entre 5 et 60 minutes. En effet, les résultats obtenus avec des temps plus longs ne sont pas meilleurs et ne justifient pas des temps de lyse plus longs.

Selon un mode avantageux, la solution d'amine quaternaire est choisie parmi une solution de chlorure d'ammonium à une concentration comprise dans une gamme allant de 100 µM à 2M, préférentiellement de 1 mM à 200mM, préférentiellement 10mM à 100mM, préférentiellement à une concentration de 20mM, et parmi une solution d'hydroxyde d'ammonium à une concentration comprise dans une gamme allant de 100 µM à 2M, préférentiellement de 1 mM à 200mM, préférentiellement 10mM à 100mM, préférentiellement à une concentration de 20mM et leurs mélanges.

Selon un mode alternatif, la solution d'amine quaternaire est préférentiellement choisie par comparaison aux résultats obtenus avec la solution d'hydroxyde d'ammonium à 20mM pendant 10 minutes et donne en particulier, lorsque testée dans les mêmes conditions, le même ratio de molécule de la MEC /ADN à plus ou moins 16%, et préférentiellement sans différence significative avec d'hydroxyde d'ammonium à 20mM pendant 10 minutes.

Selon un mode préférentiel, on choisit la concentration et le temps d'application de la solution d'amine quaternaire, préférentiellement d'ammonium par comparaison avec les résultats obtenus avec une solution de lyse d'hydroxyde d'ammonium à 20mM appliquée pendant 10 minutes. Ainsi, le ratio du résultat du dosage de la molécule de la MEC obtenus à l'étape e) sur le résultat du dosage de l'ADN dans le lysat cellulaire est comparé celui obtenu avec une solution de lyse d'hydroxyde d'ammonium à 20mM appliquée pendant le même temps.

Préférentiellement, la comparaison est effectuée par un test d'analyse statistique des variances dit One Way Anova et la concentration et le temps d'application sont choisis de telle sorte que le ratio de la solution testé ne soit pas significativement différent pour au moins p<0.05 et préférentiellement p<0.001.

L'étape de lyse est préférentiellement effectuée à température ambiant préférentiellement entre 15°C et 25°C, préférentiellement 20°C. Elle est préférentiellement menée sous agitation.

La méthode selon l'invention comprend ensuite une étape de prélèvement du lysat cellulaire.

Le lysat cellulaire est analysé. Une étape de mesure de la quantité l'ADN est effectuée afin de rationaliser les résultats de dosage des molécules de la MEC par quantité d'ADN. Cela rend ainsi possible la comparaison des résultats rapportés indirectement au nombre de cellules viables en culture. Dans le cas d'une évaluation d'un ingrédient d'intérêt cosmétique et/ou pharmaceutique en particulier, ce mode de réalisation permet ainsi d'exprimer l'effet observé sur la molécule de la MEC, par exemple une stimulation par cellule.

L'étape de mesure de la quantité d'ADN dans le lysat peut être effectuée selon les techniques habituelles en la matière par exemple un dosage biochimique à l'aide d'agent intercalant révélé par fluorescence.

Selon un mode avantageux, la molécule de la MEC étudiée et contenue dans le lysat cellulaire, éventuellement sous une forme précurseur, est dosée par méthode immunologique, préférentiellement par dosage type ELISA.

La méthode selon la présente invention comprend ensuite une étape de dosage par technique immunologique de la molécule de la MEC contenue dans la matrice extracellulaire, préférentiellement par méthode de fluorescence TRF.

Selon un mode préférentiel, les résultats de dosage de molécule de la MEC sont rationnalisés par taux d'ADN. La mesure de fluorescence peut être convertie en quantité de molécule de la MEC par le biais d'une gamme étalon de manière habituelle dans ce type de dosage.

Selon un mode préférentiel, le milieu de culture prélevé à l'étape b) et/ou le lysat cellulaire prélevé à l'étape d) sont analysés pour permet avantageusement de doser la molécule de la MEC dans les différents compartiments : intracellulaire, dans le milieu de culture et dans la MEC.

Selon un aspect, l'invention a également pour objet une méthode de dosage in vitro par technique immunologique d'au moins une molécule de la matrice extracellulaire synthétisée par des cellules en culture selon la revendication 1 comprenant au moins les étapes successives :
a) une étape de mise en culture des cellules
b) une étape de prélèvement du milieu de culture et de dosage de la molécule de la MEC et/ou un précurseur de la molécule de la MEC
c) une étape de lyse des cellules par une solution d'amine quaternaire
d) une étape de prélèvement du lysat cellulaire et de dosage de l'ADN et/ou de dosage de la molécule de la MEC et/ou un précurseur de la molécule de la MEC
e) une étape de dosage par technique immunologique de ladite molécule de la MEC contenue dans la matrice extracellulaire.

Les modes préférentiels de réalisation ont été décrits précédemment. Cette méthode permet avantageusement de doser la molécule de la MEC dans les différents compartiments : intracellulaire, dans le milieu de culture et dans la MEC.

La présente invention a également pour objet l'utilisation de la méthode de dosage selon l'invention pour le screening et/ou l'étude in vitro d'au moins un ingrédients d'intérêt cosmétique et/ou pharmaceutique pour ses propriétés à augmenter ou diminuer la quantité d'une molécule dans la matrice extracellulaire.

La présente invention a également pour objet une méthode de screening et/ou d'étude in vitro d'au moins un ingrédient d'intérêt cosmétique et/ou pharmaceutique pour ses propriétés à augmenter ou diminuer la quantité d'une molécule de la matrice extracellulaire comprenant au moins les étapes:
a) une étape de mise en culture des cellules et de culture des cellules en présence de l'ingrédient
b) préférentiellement une étape de prélèvement du milieu de culture et encore préférentiellement de dosage de la molécule de la MEC et/ou un précurseur de la molécule de la MEC dans ledit milieu de culture ainsi prélevé,
c) une étape de lyse des cellules par une solution d'amine quaternaire,
d) une étape de prélèvement du lysat cellulaire et de dosage de l'ADN et/ou de dosage de la molécule de la MEC et/ou un précurseur de la molécule de la MEC,
e) une étape de dosage par technique immunologique de ladite molécule de la MEC contenue dans la matrice extracellulaire,
et dans laquelle la culture de cellules dans le milieu de culture contenant l'ingrédient cosmétique et/ou pharmaceutique est effectuée pendant au moins 24 heures et préférentiellement de 24 à 96 heures, préférentiellement pendant 48 heures.

Selon un mode préférentiel, la méthode comprend en outre une étape f) de détermination des propriétés de l'ingrédient à augmenter ou diminuer la quantité d'une molécule de la matrice extracellulaire.

La détermination de la capacité de stimulation ou inhibition de l'ingrédient est alors effectuée par rapport à une expérience menée sans ingrédient et/ou menée avec un ingrédient dit témoin positif. Dans le cas où le collagène est la molécule de la MEC dosée, le témoin positif est préférentiellement choisi parmi la Vitamine C à une concentration qui va de 5µM à 100µM et préférentiellement à 50µM, le TGF beta préférentiellement à une dose qui va de 1ng/ml à 100ng/ml, encore préférentiellement à 10ng/ml et leurs mélanges. L'acide ascorbique et le TGF beta sont en effet des ingrédients dits « booster » du collagène et les études effectuées par la Demanderesse ont démontré leur intérêt dans la méthode de dosage selon l'invention (exemple 5).

L'ingrédient est alors classifié en fonction du ratio du dosage de la molécule de la MEC à l'étape e) rationnalisé sur l'ADN mesuré à l'étape d) par comparaison avec le ratio du dosage effectuée sans ingrédient et/ou avec un témoin positif rationnalisé sur l'ADN mesuré à l'étape d). La comparaison est effectuée préférentiellement par un test d'analyse statistique des variances dit One Way Anova.
- si le ratio obtenu de l'ingrédient est significativement supérieur à celui obtenu avec le témoin non traité pour au moins p<0.05 et préférentiellement p<0.001, alors l'ingrédient augmente la fonctionnalité de la molécule dans la MEC c'est-à-dire sa teneur dans la MEC
- si le ratio de l'ingrédient est significativement supérieur à celui obtenu avec l'acide ascorbique et/ou le TGF beta pour au moins p<0.05 et préférentiellement p<0.001, alors l'ingrédient augmente la fonctionnalité du collagène dans la MEC c'est-à-dire sa teneur dans la MEC
- si le ratio de l'ingrédient est significativement inférieur à celui du témoin non traité pour au moins p<0.05 et préférentiellement p<0.001 alors l'ingrédient diminue la fonctionnalité de la molécule dans la MEC c'est-à-dire sa teneur dans la MEC

Un ingrédient d'intérêt cosmétique ayant la capacité d'augmenter la teneur en molécules de la MEC dosées dans la MEC, en particulier pour le collagène de manière supérieure à l'acide ascorbique à 50µM peut être utilisé pour le soin cosmétique, en particulier pour le traitement anti-âge, en particulier pour diminuer les rides et les signes du vieillissement cutané, et/ou pour améliorer la fermeté de la peau.

Un ingrédient d'intérêt pharmaceutique ayant la capacité d'augmenter la teneur en molécules de la MEC dosées dans la MEC, en particulier pour le collagène de manière supérieure à la vitamine C à 50µM peut être utilisé pour améliorer le processus de cicatrisation, pour le traitement de l'arthrose.
Un ingrédient d'intérêt pharmaceutique ayant la capacité de diminuer la teneur en molécules de la MEC peut être utilisé pour le traitement des fibroses et des cicatrices hypertrophiques.

La présente invention a en outre pour objet l'utilisation d'un kit de dosage par méthode immunologique dans une méthode de dosage selon l'invention et/ou dans une méthode de screening d'un ingrédient d'intérêt cosmétique et/ou pharmaceutique selon l'invention, ledit kit comprenant une solution d'amine quaternaire et un anticorps dirigé contre la molécule de la MEC, éventuellement couplé à un anticorps secondaire.
Les caractéristiques préférentielles et/ou avantageuses de la solution d'amine quaternaire ont été précédemment décrites dans le cadre de la méthode selon l'invention.

L'anticorps dirigé contre la molécule de la MEC est un anticorps monoclonal ou polyclonal et préférentiellement choisi parmi un anticorps anti-collagène, un anticorps anti-élastine et leurs mélanges.
L'anticorps peut être mesuré, éventuellement par couplage avec un anticorps secondaire par les techniques de dosage enzyme-substrat de type EIA (Enzyme Immuno Assay), notamment ELISA (Enzyme-Linked Immunosorbent Assay), de radiolabelling dit RIA (radioimmunoassay) et par mesure de fluorescence dit FIA (Fluorescente immunoassay) en particulier par fluorescence en temps retardé dite TRF (Time Resolved fluorescence).

Selon un mode préférentiel, le kit de dosage comprend en outre une solution d'acide ascorbique à une concentration préférentielle allant de 5µM à 100µM et préférentiellement à 50µM.
Selon un mode préférentiel, le kit de dosage comprend également un anticorps dirigé contre le précurseur de la molécule de la MEC et/ou une solution de bisbenzimide pour le dosage de l'ADN dans la méthode selon l'invention.

La présente invention a également pour objet un procédé de sélection d'une solution de lyse utile dans la méthode de dosage ou selon la méthode de screening selon l'invention caractérisé en ce que la méthode de dosage selon l'invention est mise en oeuvre avec une solution de lyse à tester pendant au moins 10 min, préférentiellement 10 min, et le ratio du résultat du dosage de la molécule de la MEC obtenus à l'étape e) sur le résultat du dosage de l'ADN dans le lysat cellulaire à l'étape d) est comparé à celui obtenu avec une solution de lyse d'hydroxyde d'ammonium à 20mM appliquée pendant le même temps, préférentiellement 10 minutes.

Préférentiellement, la comparaison est effectuée par un test d'analyse statistique des variances dit One Way Anova et la solution de lyse testée est sélectionnée si le ratio de la solution testé n'est pas significativement différent pour au moins p<0.05 et préférentiellement p<0.001.

La solution de lyse ainsi sélectionnée peut être utilisée à la place de la solution d'amine quaternaire. La présente description concerne également les solutions de lyse ainsi sélectionnées selon le procédé de sélection selon l'invention.

Selon un mode de réalisation préféré, les méthodes selon l'invention sont constituées seulement des étapes a), b), c), d) e) et optionnellement f).

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, et sauf indication contraire, la température est exprimée en degré Celsius, et la pression est la pression atmosphérique, sauf indication contraire.

### EXEMPLES :

### EXEMPLE 1 : Méthode de dosage des collagènes de type I dans la MEC selon l'invention

### Principe :

La molécule de la MEC dosée par la méthode selon l'invention est le collagène de type I synthétisé par les fibroblastes.

### Protocole :

### - Etape a) :

Des fibroblastes humains normaux obtenus à partir de biopsies abdominales sont ensemencés en plaque 96 puits et cultivés dans un milieu défini (FGM) jusqu'à 100% de confluence ce qui a été obtenu après 3 jours de culture.

### - Etape b) :

Après 48 heures de culture post confluence, le milieu de culture est prélevé et éliminé.

### - Etape c) :

Une étape de lyse est effectuée : 110µl d'une solution d'hydroxyde d'ammonium à 20mM pendant 10 minutes à température ambiante et sous agitation.

### - Etape d)

Le lysat est prélevé et analysé. L'ADN double brin contenu dans le lysat est dosé par méthode au bisbenzimide (Kit Picogreen Invitrogen).

### - Etape e)

Le dosage du collagène dans la MEC est effectué directement sur la MEC par un anticorps anticollagène I. Un anticorps secondaire couplé à l'europium a été utilisé avec une solution de révélation et la fluorescence a été mesurée. (kit Delfia® - Perkin Elmer).

Les résultats de fluorescence ont été rationnalisés par rapport au taux d'ADNdb mesuré.

L'expérience a été effectuée sur 6 puits (n=6)

Les résultats présentés correspondent à la moyenne (Moy) et l'écart type (EC)

### Résultats :

| | | |
|---|---|---|
| Coll I (RFU) | Moy | 104228 |
| | EC | 4757 |
| ADN (ng/ml) | Moy | 303.01 |
| | EC | 10.98 |
| RATIO Coll I /ADN | Moy | 343.98 |
| | EC | 12.79 |

### Discussions :

La quantité de fluorescence étant proportionnelle à la quantité de collagène mesurée, la quantité de collagène peut être déterminée à l'aide d'une gamme étalon.

### EXEMPLE 2 : Etude de l'influence du temps de lyse sur la méthode de dosage du collagène de type I dans la MEC selon l'invention

### Principe :

La molécule de la MEC dosée par la méthode selon l'invention est le collagène de type I synthétisé par les fibroblastes en fonction du temps de lyse.

### Protocole :

Le protocole décrit dans l'exemple 1 est appliqué avec différentes durées de l'étape de lyse avec la solution d'hydroxyde d'ammonium 20mM allant de 5 minutes à 240 minutes.

L'expérience est effectuée pour chaque durée sur 6 puits (n=6)

Les résultats présentés correspondent à la moyenne (Moy) et l'écart type (EC) et la significativité déterminée par analyse One way Anova (Dunnett) au regard de la durée optimum de 10 minutes.
NS : non significatif NT : non analysable
* : significatif à p<0,05 ** : significatif à p<0.01 *** : significatif à p<0.001

### Résultats :

**Tableau 2**

| Temps (en min) | | 2'30 | 5 | 7'30 | 10 | 15 | 30 | 60 | 120 |
|---|---|---|---|---|---|---|---|---|---|
| Coll I (RFU) | Moy | 44319 | 46031 | 48762 | 50351 | 49301 | 51632 | 53188 | 52667 |
| | EC | 2416 | 2436 | 1492 | 1377 | 2224 | 3404 | 3830 | 4143 |
| | Dunnett | * | NS | NS | - | NS | NS | NS | NS |
| ADNdb (ng/mL) | Moy | 155,11 | 291,35 | 363,72 | 430,25 | 438,66 | 412,30 | 417,94 | 422,42 |
| | T-test | 26,26 | 46,95 | 15,16 | 39,21 | 28,57 | 15,85 | 15,59 | 17,44 |
| | Dunnett | ** | ** | ** | - | NS | NS | NS | NS |
| Ratio= Coll/ ADNdb | Moy | 295,57 | 161,88 | 134,18 | 117,86 | 112,87 | 125,31 | 127,22 | 124,89 |
| | EC | 70,62 | 30,23 | 4,91 | 11,29 | 10,11 | 8,13 | 7,07 | 11,68 |
| | Dunnett | * | NS | NS | - | NS | NS | NS | NS |

### Conclusion :

Il est observé qu'à 2.5 minutes, l'ADN extrait est en quantité trop faible pour être révélateur de la quantité de cellules viables. A partir de 5 minutes, la quantité de collagène mesurée et la quantité d'ADNdb sont homogènes au cours du temps. Le collagène n'est pas dégradé par la solution d'hydroxyde d'ammonium au cours du temps et la durée optimale de lyse est déterminée à 10 minutes. Des expériences menées pendant des durées d'étape de lyse allant jusqu'à 48 heures ont été effectuées et ont confirmé ces observations.

### EXEMPLE 3 : Etude de l'influence de la nature de la solution de lyse sur la méthode de dosage du collagène de type V dans la MEC selon l'invention

### Principe :

La molécule de la MEC dosée par la méthode selon l'invention est le collagène de type V synthétisé par les fibroblastes et l'étude est menée en fonction de la nature de la solution de lyse.

### Protocole :

Le protocole décrit dans l'exemple 1 est appliqué de la même manière.

Ainsi des fibroblastes humains normaux obtenus à partir de biopsies abdominales sont ensemencés en plaque 96 puits et cultivés dans un milieu défini (FGM) jusqu'à 100% de confluence.

Après 48 heures de culture post confluence à 37°C sous 0.5% de CO₂, le milieu de culture est prélevé.

Les solutions de lyse testées sont les suivantes :
- solution selon l'invention NH4OH : 110µl d'une solution d'hydroxyde d'ammonium à 20mM.
- solution 1 : solution contenant 52,55 g d'urée, 15,01 g de thiourée, 964 mg de DTT et 37,2 mg EDTA
- solution 2 : solution contenant Tris 10mM/Triton 0.1%
- solution 3 : solution contenant Hepes 50mM
- solution 4 : solution contenant du Tris HCl à 50Mm pH7.5), un agent détergent (sodium de deoxycholate à 0.5mM) et un agent chelatant (EGTA à 20mM)
- solution 5 : solution contenant EDTA 50mM
- solution 6 : solution contenant NaOH 0.2N et 1%SDS

Le temps d'application de la solution de lyse testée est 10 minutes à température ambiante et sous agitation.

Le lysat est prélevé et analysé. L'ADN double brin contenu dans le lysat est dosé par méthode au bisbenzimide (Kit Picogreen Invitrogen).

Le dosage du collagène V dans la MEC est effectué directement sur la MEC par un anticorps anticollagène V. Un anticorps secondaire couplé à l'europium a été utilisé avec une solution de révélation et la fluorescence a été mesurée. (kit Delfia® - Perkin Elmer).

La fluorescence est mesurée.

L'expérience est effectuée pour chaque solution de lyse sur 6 puits (n=6)

Les résultats sont présentés dans le tableau 3.1

L'expérience est renouvelée et les solutions de lyse testées sont les suivantes :
- solution selon l'invention NH4OH : 110µl d'une solution d'hydroxyde d'ammonium à des concentrations comprises entre 200µM et 2M .
- Solution de monoéthylamine à 20mM
- Solution de diéthylamine à 20mM
- Solution de triéthylamine à 20mM

Les résultats sont présentés dans le tableau 3.2 et 3.3

Les résultats présentés correspondent à la moyenne (Moy) et l'écart type (EC) et la significativité déterminée par analyse One way Anova (Dunnett) au regard de la solution de lyse NH4OH.
NS : non significatif NT : non analysable
* : significatif à p<0,05 ** : significatif à p<0.01 *** : significatif à p<0.001

### Résultats :

**Tableau 3.1 :**

| Solution | | NH4OH | 1 | 2 | 3 | 4 | 5 | 6 |
|---|---|---|---|---|---|---|---|---|
| Coll V (RFU) | Moy | 60881 | 61017 | 60947 | 50135 | 53508 | 46558 | 13513 |
| | EC | 2948 | 3703 | 2166 | 1768 | 3063 | 5972 | 4489 |
| ADN | Moy | 316.27 | 501.08 | 155.80 | 23.50 | 156.22 | 5.80 | 0 |
| | EC | 21.51 | 128.84 | 13.47 | 7.48 | 5.68 | 3.85 | 0.18 |
| Ratio : Coll/ ADN | Moy | 193,13 | 126,61 | 388,64 | 2342 | 342,76 | 10447 | 0 |
| | EC | 14,57 | 30,62 | 23,07 | 856 | 21,40 | 5080 | 0 |
| | Anova (dunnett) | - | ** | ** | NT | ** | NT | NT |

**Tableau 3.2 :**

| Solution | | NH4OH 20µM | Monoéthylamine | Diéthylamine | Triéthylamine |
|---|---|---|---|---|---|
| Coll V (RFU) | Moy | 104228 | 67663 | 46155 | 54680 |
| | EC | 4757 | 4044 | 1849 | 3176 |
| ADN | Moy | 303.01 | 827.11 | 774.15 | 752.26 |
| | EC | 10.98 | 185.49 | 31.20 | 60.70 |
| Ratio : Coll/ADN | Moy | 343.98 | 81.81 | 59.62 | 72.69 |
| | EC | 12.79 | 20 | 4.31 | 7.16 |
| | % | 100 | 23,78 | 17,33 | 21,13 |
| | Anova (dunnett) | - | *** | *** | *** |

**Tableau 3.3 :**

| Solution | | NH4OH | | | | |
|---|---|---|---|---|---|---|
| | | 200 µM | 2 mM | 20 mM | 200 mM | 2 M |
| Coll V (RFU) | Moy | 114177 | 117380 | 104228 | 118318 | 119292 |
| | EC | 5889 | 6187 | 4757 | 6504 | 2839 |
| ADN | Moy | 282,65 | 259,39 | 303,01 | 397,80 | 512,48 |
| | EC | 21,05 | 25,23 | 10,98 | 41,95 | 34,27 |
| Ratio : Coll/ ADN | Moy | 403,95 | 452,53 | 343,98 | 297,43 | 232,77 |
| | EC | 41,81 | 77,75 | 12,79 | 41,73 | 17,50 |
| | % | 117,43 | 131,56 | 100,00 | 86,47 | 67,67 |
| | Anova (dunnett) | ns | ns | - | ns | ns |

### Discussions :

Les tests effectués mettent en évidence que la solution d'hydroxyde d'ammonium permet d'obtenir des résultats plus fiables, répétables et reproductibles que les solutions classiques de lyse cellulaire et que les solutions d'amine primaire, secondaire et tertiaire. La solution d'hydroxyde d'ammonium permet des dosages dans des conditions maximales pour à la fois la lyse cellulaire et la mesure du collagène V. Les autres solutions d'amine quaternaire testées ont montrées des résultats également très intéressants à la fois la lyse cellulaire et la mesure du collagène V. Par ailleurs, les ratios collagène V/ADN obtenus avec différentes concentrations de solution de lyse d'hydroxyde d'ammonium ne sont pas significativement différents de celui obtenu avec une solution à 20Mm. Cette concentration solution est optimale du fait de sa praticité d'utilisation.

### EXEMPLE 4 : Méthode de dosage de collagène de type I dans les trois compartiments selon l'invention

### Principe :

Les différents compartiments : intracellulaire (lysat cellulaire), milieu de culture et MEC sont récupérés et les différentes formes du collagène I sont dosées.

### Protocole :

La méthode décrite dans l'exemple 1 est appliquée et les milieux de culture et de lyse prélevés sont analysés.

L'expérience est effectuée sans ingrédient actif d'un part et avec l'acide ascorbique solubilisé dans du PBS à différentes concentrations de 0.1 à 100µM. Sur le milieu de culture prélevé, un dosage de la quantité de procollagène est effectué (kit PIPc, TAKARA,) par ELISA pour déterminer la quantité de procollagène extracellulaire (tableau 4.1)

Sur le milieu de lyse prélevé, un dosage de la quantité de procollagène (kit PIPc, TAKARA,) par ELISA est effectué pour déterminer la quantité de procollagène intracellulaire (tableau 4.2). Un dosage de la quantité d'ADN (kit Picogreen, invitrogen) est également effectué pour rationnaliser l'ensemble des mesures dans chaque puits à la quantité de cellules viables.

Le dosage de la quantité de collagène I dans la MEC est effectué et présenté dans le tableau 4.3

Les quantités de collagène et procollagène mesurées sont rapportées à la quantité d'ADN présente dans chaque puits.

Une analyse statistique One Way Anova a été effectuée par rapport au témoin.
NS : non significatif NT : non analysable
* : significatif à p<0,05 ** : significatif à p<0.01 *** : significatif à p<0.001

### Résultats :

**Tableau 4.1 :**

| Ingrédient actif | | témoin | Acide ascorbique (µM) | | | | |
|---|---|---|---|---|---|---|---|
| | | | 0.1 | 1 | 10 | 50 | 100 |
| Procoll ng/ml | Moy | 2104 | 1987 | 1983 | 2756 | 2852 | 2845 |
| ADN | Moy | 340 | 318 | 303 | 281 | 279 | 291 |
| Ratio : Procoll / ADN | Moy | 6,22 | 6,26 | 6,57 | 9,83 | 10,25 | 9,78 |
| Stat | Moy | 100,00 | 100,75 | 105,74 | 158,16 | 164,89 | 157,30 |
| | EC | 7,15 | 4,74 | 9,07 | 12,22 | 12,01 | 9,26 |
| | Dunnett | - | ns | ns | ** | ** | ** |

**Tableau 4.2 :**

| Ingrédient actif | | témoin | Acide ascorbique (µM) | | | | |
|---|---|---|---|---|---|---|---|
| | | | 0.1 | 1 | 10 | 50 | 100 |
| Procoll ng/ml | Moy | 117 | 116 | 108 | 114 | 60 | 66 |
| ADN | Moy | 340 | 318 | 303 | 281 | 279 | 291 |
| Ratio : Procoll /ADN | Moy | 0,34 | 0,37 | 0,36 | 0,41 | 0,22 | 0,23 |
| Stat | Moy | 100,00 | 102,85 | 100,76 | 108,02 | 60,83 | 63,69 |
| | EC | 6,05 | 4,84 | 9,80 | 10,65 | 7,74 | 8,83 |
| | Dunnett | - | ns | ns | ns | ** | ** |

**Tableau 4.3 :**

| Ingrédient actif | | témoin | Acide ascorbique (µM) | | | | |
|---|---|---|---|---|---|---|---|
| | | | 0.1 | 1 | 10 | 50 | 100 |
| Coll I | Moy | 75204 | 74156 | 74419 | 78694 | 79146 | 79796 |
| ADN | Moy | 340 | 318 | 303 | 281 | 279 | 291 |
| Ratio : Coll I/ ADN | Moy | 221,83 | 233,65 | 246,46 | 280,82 | 284,31 | 273,96 |
| Stat | Moy | 100,00 | 105,33 | 111,10 | 126,59 | 128,17 | 123,50 |
| | EC | 3,92 | 3,61 | 6,19 | 6,82 | 7,86 | 4,72 |
| | Dunnett | - | ns | ** | ** | ** | ** |

### Discussions :

La quantité de procollagène et de collagène peut être calculée en se rapportant à une courbe étalon respectivement de procollagène et collagène humain L'acide ascorbique ou vitamine C est connu pour augmenter la synthèse et la sécrétion de procollagène dans le milieu extracellulaire ce que démontre également. Ces effets de stimulation sont également mesurés avec la méthode selon l'invention, y compris au niveau de la teneur en collagène I dans la MEC. Cet effet est dose-dépendant jusqu'à 50µM où l'effet seuil de la molécule est observé. La concentration observée dans le milieu intracellulaire sous l'effet de la vitamine C est inférieure à celle observée avec le témoin non traité dans la mesure où le procollagène a été sécrété dans le milieu extracellulaire de manière plus importante.

### EXEMPLE 5 : Utilisation de la méthode de dosage de collagène de type I et de type V comme méthode de screening :

### Principe :

Des ingrédients actifs d'intérêt cosmétique connus pour leur effet stimulant de la synthèse de collagène sont testés pour leur propriété sur le collagène de type I dans la MEC puis sur le collagène de type V dans la MEC.

### Protocole :

L'expérience est effectuée selon le protocole décrit à l'exemple 1 à partir fibroblastes extraits d'une biopsie d'un donneur âgé de 63 ans.

Les ingrédients actifs d'intérêt cosmétique qui ont été testés sont :
- Acide ascorbique 50µM solubilisée dans une solution tampon PBS est testé à différentes concentrations de 5 à 100 µM.
- un hydrolysat de protéine de soja décrit dans la demande de brevet WO2009121422 et commercialisé par la Demanderesse sous le nom de Phytokine™ est testé à différentes concentrations de 0.5 à 2% v/v

Les cellules à confluence ont été incubées en présence des ingrédients actifs pendant 48heures à 37°C sous 0.5% de CO₂ ou sans ingrédient actif (témoin) pendant 48heures à 37°C sous 0.5% de CO₂

L'expérience est effectuée pour chaque condition sur 6 puits (n=6) - Le résultat présenté correspond à la moyenne (Moy) et l'écart type (EC)

Les résultats obtenus pour le dosage du collagène de type I sous l'effet de Phytokine™ sont présentés dans le tableau 5.1 et ceux pour le dosage du collagène de type V dans le tableau 5.2.

Les résultats obtenus pour le dosage du collagène de type I sous l'effet de l'acide ascorbique sont présentés dans le tableau 5.3 et ceux pour le dosage du collagène de type V dans le tableau 5.4.

La significativité est calculée par test One Way Anova (Dunnett) par rapport au témoin.
NS : non significatif NT : non analysable
* : significatif à p<0,05 ** : significatif à p<0.01 *** : significatif à p<0.001

### Résultats :

**Tableau 5.1 :**

| Ingrédient actif | | Témoin | Phytokine ™ (% v/v) | | | | |
|---|---|---|---|---|---|---|---|
| | | | 0.5 | 1 | 1.25 | 1.5 | 2 |
| Coll I (RFU) | Moy | 78857 | 86149 | 81474 | 84256 | 82888 | 85938 |
| | EC | 793 | 1496 | 1806 | 3592 | 2868 | 2093 |
| ADN | Moy | 282.67 | 284.89 | 271.07 | 272.17 | 260.96 | 254.26 |
| | EC | 6.8 | 7.0 | 4.8 | 7.6 | 6.9 | 8.0 |
| Ratio : Coll/ADN | Moy | 277.54 | 302.06 | 300.42 | 307.51 | 315.94 | 341.07 |
| | EC | 10.4 | 1.8 | 16.2 | 25.3 | 19.4 | 15.6 |
| Versus Témoin | Moy | 100 | 108.84 | 108.25 | 110.80 | 112.98 | 122.89 |
| | EC | 2.62 | 4.09 | 3.28 | 6.00 | 4.23 | 5.22 |
| | Dunnett | - | * | * | * | * | * |

**Tableau 5.2 :**

| Ingrédient actif | | Témoin | Phytokine ™ (% v/v) | | | | |
|---|---|---|---|---|---|---|---|
| | | | 0.5 | 1 | 1.25 | 1.5 | 2 |
| Coll V (RFU) | Moy | 13479 | 13483 | 14113 | 14451 | 15452 | 16818 |
| | EC | 560 | 1096 | 870 | 593 | 751 | 716 |
| ADN | Moy | 268.68 | 259.81 | 250.56 | 250.72 | 247.90 | 222.37 |
| | EC | 7.9 | 5.2 | 7.7 | 7.7 | 5.5 | 8.6 |
| Ratio : Coll/ADN | Moy | 49.9 | 51.86 | 56.29 | 57.80 | 62.38 | 72.52 |
| | EC | 1.4 | 5.3 | 1.5 | 3.0 | 2.5 | 6.7 |
| Versus Témoin | Moy | 100 | 103.92 | 112.80 | 115.84 | 125.01 | 151.34 |
| | EC | 6.3 | 9.84 | 10.68 | 7.72 | 8.75 | 10.69 |
| | Dunnett | - | NS | NS | * | * | * |

**Tableau 5.3 :**

| Ingrédient actif | | Témoin | Acide ascorbique (µM) | | | | |
|---|---|---|---|---|---|---|---|
| | | | 5 | 10 | 25 | 50 | 100 |
| Coll I (RFU) | Moy | 52511 | 59802 | 59569 | 59088 | 54454 | 50765 |
| | EC | 3160,78 | 3278,42 | 2991,90 | 1412,56 | 5177,36 | 5084,09 |
| ADN | Moy | 242 | 220 | 249 | 232 | 180 | 204 |
| | EC | 17,34 | 23,70 | 9,28 | 13,62 | 30,42 | 25,35 |
| Ratio : Coll/ADN | Moy | 217,51 | 273,40 | 239,53 | 254,84 | 309,87 | 249,31 |
| | EC | 12,52 | 14,74 | 7,88 | 13,00 | 66,93 | 9,50 |
| Versus Témoin | Moy | 100,00 | 125,69 | 110,12 | 117,16 | 130,66 | 114,62 |
| | EC | 5,36 | 7,29 | 3,70 | 6,44 | 10,30 | 4,09 |
| | Dunnett | - | * | * | * | * | * |

**Tableau 5.4 :**

| Ingrédient actif | | Témoin | Acide ascorbique (µM) | | | | |
|---|---|---|---|---|---|---|---|
| | | | 5 | 10 | 25 | 50 | 100 |
| Coll V (RFU) | Moy | 12337 | 12644 | 14127 | 16172 | 16230 | 17994 |
| | EC | 1103,62 | 734,56 | 1072,20 | 971,01 | 1125,26 | 460,87 |
| ADN | Moy | 241 | 212 | 241 | 236 | 236 | 246 |
| | EC | 11,90 | 12,22 | 8,57 | 3,51 | 6,75 | 5,67 |
| Ratio : Coll/ADN | Moy | 51,07 | 59,83 | 58,76 | 68,69 | 68,86 | 73,12 |
| | EC | 2,40 | 4,56 | 6,39 | 5,29 | 5,32 | 2,83 |
| Versus Témoin | Moy | 100,00 | 117,16 | 115,06 | 134,49 | 134,82 | 143,17 |
| | EC | 6,10 | 8,17 | 11,46 | 9,63 | 9,32 | 5,08 |
| | Dunnett | - | * | * | * | * | * |

### Discussions :

Les résultats montrent que Phytokine™ induit une stimulation de synthèse de collagène I et V avec un effet dose et de façon significative de même que l'acide ascorbique. Pour le dosage du collagène I la dose optimale d'efficacité de l'acide ascorbique est 50µM.

### EXEMPLE 6 : Utilisation de la méthode de dosage de collagène de type I comme méthode de screening

### Principe :

Des ingrédients actifs d'intérêt cosmétique connus pour leur effet stimulant de la synthèse de collagène sont testés pour leur propriété sur le collagène de type I dans la MEC.

### Protocole :

L'expérience est effectuée selon le protocole décrit à l'exemple 1 à partir fibroblastes extraits d'une biopsie d'un donneur âgé de 63 ans.

Les ingrédients actifs d'intérêt cosmétique qui ont été testés sont :
- acide ascorbique 50µM solubilisée dans une solution tampon PBS
- extrait végétal testé à 1% (v/v) qui est un extrait aqueux de feuilles de Davilla rugosa

Les cellules à confluence ont été incubées en présence des ingrédients actifs pendant 48heures à 37°C sous 0.5% de CO₂ ou sans ingrédient actif (témoin) pendant 48heures à 37°C sous 0.5% de CO₂

L'expérience est effectuée pour chaque condition sur 6 puits (n=6) - Le résultat présenté correspond à la moyenne (Moy) et l'écart type (EC)

La significativité est calculée par test One Way Anova (Dunnett)
NS : non significatif NT : non analysable
* : significatif à p<0,05 ** : significatif à p<0.01 *** : significatif à p<0.001

### Résultats :

| Ingrédient actif | | Témoin | Acide ascorbique (50µM) | extrait végétal 1% (v/v) |
|---|---|---|---|---|
| Coll I (RFU) | Moy | 153536 | 169132 | 263664 |
| | EC | 9729 | 11038 | 9440 |
| ADN | Moy | 539,4 | 497,4 | 384,6 |
| | EC | 22,1 | 26,4 | 16,5 |
| Ratio : Coll/ADN | Moy | 284,86 | 340,49 | 687,29 |
| | EC | 18,16 | 22,11 | 52,09 |
| Versus Témoin | Moy | 100,00 | 119,53 | 241,27 |
| | EC | 6,38 | 7,76 | *18,29* |
| | Dunnett | - | ** | * |

### Discussions :

Les résultats montrent que l'acide ascorbique à 50µM et l'extrait végétal testé induisent une augmentation de la quantité de collagène I dans la MEC de façon significative. L'extrait végétal testé est plus efficace que l'acide ascorbique et est donc un ingrédient d'intérêt cosmétique et/ou pharmaceutique utilisable notamment pour le traitement cosmétique des rides et pour augmenter la fermeté cutanée.

### EXEMPLE 7 : Etude des propriétés d'ingrédients screenés avec la méthode selon l'invention

### - dosage de procollagène dans le milieu de culture :

Lors de l'expèrience 6 le procollagène a été dosé dans le milieu de culture prélevé à l'étape b) conformément dans la méthode décrite l'exemple 4.

Les résultats sont présentés dans le tableau 7.

| Ingrédient actif | | Témoin | Acide ascorbique (50µM) | extrait végétal 1% (v/v) |
|---|---|---|---|---|
| Procoll (ng/ml) | Moy | 1175 | 1983 | 624 |
| | EC | 156 | 400 | 18.5 |
| ADN | Moy | 539 | 497.3 | 384.6 |
| | EC | 22 | 26.41 | 16.5 |
| Ratio : ProColl/ADN | Moy | 2.18 | 4.01 | 1.62 |
| | EC | 0.33 | 0.9 | 0.08 |
| Versus Témoin | Moy | 100 | 171.17 | 74.35 |
| | EC | 14.98 | 31.53 | 3.5 |
| | Dunnett | - | *** | NS |

*Conclusion :* L'extrait végétal ne démontre pas d'augmentation de la synthèse de procollagène dans le milieu de culture mais avait démontré une augmentation de la quantité de collagène I dans la matrice.

### - analyse immunohistochimique

Protocole : Une analyse immunohistochimique a été effectuée selon le protocole suivant :
Les fibroblastes humains normaux issus d'une biopsie ont été ensemencées sur des lames labtecks 8 puits à raison de 40000 cellules/cm2 et cultivées en milieu de culture défini FGM jusqu'à 100% de confluence. Les ingrédients évalués, l'extrait végétal 1% v/v ou acide ascorbique 50µM ont été appliqués pendant 72h. Après 72H de culture post confluence, le milieu de culture est éliminé et l'immunomarquage est réalisé. L'anticorps primaire anti-collagène I polyclonal est appliqué puis après élimination du milieu l'anticorps secondaire couplé à un fluorochrome alexa 488 est rajouté.

L'observation de la fluorescence est réalisée à l'aide du microscope confocal LSM700.

*Résultats :* Les résultats mesurés en fonction de la fluorescence sont les suivants :

| Ingrédient actif | | Témoin | Acide ascorbique (50µM) | extrait végétal 1% (v/v) |
|---|---|---|---|---|
| Aire /cellules | Moy | 1871.01 | 124238.13 | 217414.26 |
| | EC | 8426.84 | 41013.16 | 47249.63 |
| | % | 100 | 114.96 | 201.18 |

### Conclusion :

L'étude de visualisation a permis de confirmer les résultats obtenus avec la méthode selon l'invention.

Ces résultats démontrent par ailleurs l'intérêt de screener un ingrédient en fonction de ses propriétés sur la quantité de molécules dans la MEC et justifient l'intérêt de la méthode selon l'invention.

## Revendications

1. Méthode de dosage *in vitro* par technique immunologique d'au moins une molécule de la matrice extracellulaire (MEC) synthétisée par des cellules en culture comprenant au moins les étapes :
a) une étape de mise en culture des cellules à confluence pendant au moins 24 heures,
b) une étape préférentielle de prélèvement du milieu de culture,
c) une étape de lyse des cellules par une solution d'amine quaternaire,
d) une étape de prélèvement du lysat cellulaire et de dosage d'ADN et/ou de ladite molécule de la MEC dans le lysat cellulaire,
e) une étape de dosage par technique immunologique de ladite molécule de la MEC dans la matrice extracellulaire,
et dans laquelle les cellules en culture sont maintenues en culture au stade de confluence pendant une durée qui va de 24 à 96 heures avant la réalisation de l'étape c).

2. Méthode de dosage selon la revendication 1 dans laquelle l'étape b) de prélèvement du milieu de culture est effectuée.

3. Méthode de dosage selon l'une des revendications précédente dans laquelle la molécule de la MEC est choisie parmi les protéines constitutives de la MEC, les glycoprotéines constitutives de la MEC, les glycoaminoglycanes constitutives de la MEC, les protéoglycanes constitutives de la MEC et les facteurs de croissances contenues dans la MEC, et avantageusement choisie parmi les collagènes de type I, III, V, VI, XII, XIV, XVI, IV et VII, l'élastine, la tropoélastine, la fibrilline 1, LOX, LOXL, l'EBP (Elastine Binding Protein), les fibullines 3 et 5, les Emilin 1 et 2, le Perlecan, le versican, la decorine, le biglycan, le lumican, la fibronectine, la laminine, l'acide hyaluronique, heparane sulfate, le VEGF, le PDGF, le FGF-2, FGF-7, et HGF.

4. Méthode de dosage selon l'une des revendications précédentes dans laquelle les cellules en culture sont choisies parmi sont les cellules stromales, préférentiellement les fibroblastes, les ostéoblastes et/ou adipoblastes, épithéliales, préférentiellement les kératinocytes ou les cellules endothéliales, et avantageusement choisies parmi des fibroblastes et/ou des kératinocytes et/ou adipocytes.

5. Méthode de dosage selon l'une des revendications 1 à 4 dans laquelle un ingrédient actif d'intérêt cosmétique et/ou pharmaceutique est ajouté dans le milieu de culture, et avantageusement choisi parmi la vitamine C, préférentiellement à une dose allant de 5µM à 100µM, et le TGF beta, préférentiellement à une dose allant de 1 ng/ml à 100ng/ml.

6. Méthode de dosage selon l'une des revendications 2 à 5 dans laquelle la molécule de la MEC et/ou un précurseur de la molécule de la MEC est dosée par méthode immunologique dans le milieu de culture prélevé à l'étape b), préférentiellement par dosage type ELISA.

7. Méthode de dosage selon l'une des revendications précédentes dans laquelle la solution d'amine quaternaire est une solution d'ammonium, éventuellement sous forme de sels, et avantageusement choisie parmi une solution de chlorure d'ammonium, une solution d'hydroxyde d'ammonium et leurs mélanges.

8. Méthode de dosage selon l'une des revendications précédentes dans laquelle la solution d'amine quaternaire est à une concentration allant de 10mM à 200Mm.

9. Méthode de dosage selon l'une des revendications précédentes dans laquelle la solution d'amine quaternaire est appliquée sur les cellules pendant un temps allant de 5 à 60 minutes.

10. Méthode de dosage selon l'une des revendications précédentes dans laquelle l'étape e) de dosage par technique immunologique de ladite molécule contenue dans la matrice extracellulaire est effectuée par méthode de fluorescence TRF.

11. Méthode de dosage selon l'une des revendications précédentes comprenant au moins les étapes:
a) une étape de mise en culture des cellules, et préférentiellement culture des cellules à confluence pendant au moins 24 heures,
b) une étape de prélèvement du milieu de culture et de dosage de la molécule de la MEC et/ou un précurseur de la molécule de la MEC,
c) une étape de lyse des cellules par une solution d'amine quaternaire,
d) une étape de prélèvement du lysat cellulaire et de dosage de l'ADN et/ou de dosage de la molécule de la MEC et/ou un précurseur de la molécule de la MEC
e) une étape de dosage par technique immunologique de ladite molécule de la MEC contenue dans la matrice extracellulaire.

12. Utilisation de la méthode de dosage selon l'une des revendications précédentes pour le screening et/ou l'étude *in vitro* d'au moins un ingrédient d'intérêt cosmétique et/ou pharmaceutique pour ses propriétés à augmenter ou diminuer la quantité d'une molécule dans la matrice extracellulaire.

13. Méthode de screening et/ou d'étude *in vitro* d'au moins un ingrédient d'intérêt cosmétique et/ou pharmaceutique pour ses propriétés à augmenter ou diminuer la quantité d'une molécule dans la matrice extracellulaire comprenant au moins les étapes :
a) une étape de mise en culture des cellules en présence de l'ingrédient,
b) préférentiellement une étape de prélèvement du milieu de culture et encore préférentiellement de dosage de la molécule de la MEC et/ou un précurseur de la molécule de la MEC dans ledit milieu de culture ainsi prélevé,
c) une étape de lyse des cellules par une solution d'amine quaternaire,
d) une étape de prélèvement du lysat cellulaire et de dosage de l'ADN et/ou de dosage de la molécule de la MEC et/ou un précurseur de la molécule de la MEC,
e) une étape de dosage par technique immunologique de ladite molécule de la MEC contenue dans la matrice extracellulaire,
et dans laquelle la culture de cellules dans le milieu de culture contenant l'ingrédient cosmétique et/ou pharmaceutique est effectuée pendant au moins 24 heures.

14. Utilisation d'un kit de dosage par méthode immunologique dans une méthode de dosage selon l'une des revendications 1 à 11 ou dans une méthode de screening selon la revendication 13, ledit kit comprenant une solution d'amine quaternaire et un anticorps dirigé contre la molécule de la MEC, éventuellement couplé à un anticorps secondaire.

15. Procédé de sélection d'une solution de lyse utile dans la méthode de dosage selon l'une des revendications 1 à 11 ou selon la méthode de screening selon la revendication 13, **caractérisé en ce que** la méthode de dosage est mise en oeuvre avec une solution de lyse à tester pendant au moins 10 min, préférentiellement 10 min, et le ratio du résultat du dosage de la molécule de la MEC obtenus à l'étape e) sur le résultat du dosage de l'ADN dans le lysat cellulaire à l'étape d) est comparé à celui obtenu avec une solution de lyse d'hydroxyde d'ammonium à 20mM appliquée pendant le même temps, préférentiellement 10 minutes.

## Patentansprüche

1. Verfahren zum In-vitro-Dosieren mindestens eines Moleküls der extrazellulären Matrix (MEC), das durch Zellen in Kultur synthetisiert ist, mittels immunologischer Technik, umfassend mindestens die folgenden Schritte:
a) einen Schritt der Kultivierung der Zellen in Konfluenz für mindestens 24 Stunden,
b) einen bevorzugten Schritt der Entnahme des Kulturmediums,
c) einen Schritt der Lyse der Zellen mittels einer quaternären Aminlösung,
d) einen Schritt der Entnahme des Zelllysats und der Dosierung einer DNA und/oder des Moleküls der MEC in dem Zelllysat,
e) einen Schritt der Dosierung des Moleküls der MEC in der extrazellulären Matrix durch immunologische Technik,
und bei dem die Zellen in Kultur im Konfluenzstadium in Kultur für eine Dauer gehalten werden, die von 24 bis 96 Stunden vor der Durchführung des Schrittes c) reicht.

2. Verfahren zum Dosieren gemäß Anspruch 1, bei dem der Schritt b) der Entnahme des Kulturmediums durchgeführt wird.

3. Verfahren zum Dosieren gemäß einem der vorhergehenden Ansprüche, bei dem das Molekül der MEC unter den die MEC bildenden Molekülen, den die MEC bildenden Glycoproteinen, den die MEC bildenden Glycoaminoglycanen, den die MEC bildenden Proteoglycanen und den in der MEC enthaltenen Wachstumsfaktoren ausgewählt ist, und vorzugsweise unter den Kollagenen des Typs I, III, V, VI, XII, XIV, XVI, IV und VII, dem Elastin, dem Tropoelastin, dem Fibrillin 1, LOX, LOXL, EBP (Elastine Binding Protein), den Fibullinen 3 und 5, den Emillin 1 und 2, dem Perlecan, Versican, Decorin, Biglycan, Lumican, Fibronectin, Laminin, der Hyaluronsäure, dem Haparansulfat, VEGF, PDGF, FGF-2, FGF-7 und HGF ausgewählt ist.

4. Verfahren zum Dosieren gemäß einem der vorhergehenden Ansprüche, bei dem die Zellen in Kultur unter den Stromazellen, vorzugsweise den Fibroblasten, den Osteoblasten und/oder Adipoblasten, Epithelzellen, vorzugsweise den Keratinocyten oder den Endothelzellen, ausgewählt sind, und vorteilhafterweise unter den Fibroblasten und/oder Keratinocyten und/oder Adipocyten ausgewählt sind.

5. Verfahren zum Dosieren gemäß einem der Ansprüche 1 bis 4, bei dem ein aktiver Wirkstoff von kosmetischem und/oder pharmazeutischem Interesse zu dem Kulturmedium hinzugefügt wird, vorteilhafterweise ausgewählt unter Vitamin C, vorzugsweise in einer Dosis von 5 µM bis 100 µM, und TGF-Beta, vorzugsweise in einer Dosis von 1 ng/ml bis 100 ng/ml.

6. Verfahren zum Dosieren gemäß einem der Ansprüche 2 bis 5, bei dem das Molekül der MEC und/oder ein Vorläufer des Moleküls der MEC durch ein immunologisches Verfahren in dem in Schritt b) entnommenen Kulturmedium, vorzugsweise durch Dosierung des Typs ELISA, dosiert wird.

7. Verfahren zum Dosieren gemäß einem der vorhergehenden Ansprüche, bei dem die quaternäre Aminlösung eine Ammoniumlösung, eventuell in Form von Salzen, ist, und vorteilhafterweise unter einer Ammoniumchloridlösung, einer Ammoniumhydroxidlösung und ihren Mischungen ausgewählt ist.

8. Verfahren zum Dosieren gemäß einem der vorhergehenden Ansprüche, bei dem die quaternäre Aminlösung in einer Konzentration von 10 mM bis 200 mM vorhanden ist.

9. Verfahren zum Dosieren gemäß einem der vorhergehenden Ansprüche, bei der die quaternäre Aminlösung auf die Zellen für eine Zeit von 5 bis 60 Minuten angewandt wird.

10. Verfahren zum Dosieren gemäß einem der vorhergehenden Ansprüche, bei dem der Schritt e) der Dosierung durch immunologische Technik des in der extrazellulären Matrix enthaltenen Moleküls durch Fluoreszenzmethode TRF durchgeführt wird.

11. Verfahren zum Dosieren gemäß einem der vorhergehenden Ansprüche, umfassend mindestens die folgenden Schritte:
a) einen Schritt der Kultivierung der Zellen, und vorzugsweise Kultivierung der Zellen in Konfluenz für mindestens 24 Stunden,
b) einen Schritt der Entnahme des Kulturmediums und der Dosierung des Moleküls der MEC und/oder eines Vorläufers des Moleküls der MEC,
c) einen Schritt der Lyse der Zellen mittels einer quaternären Aminlösung,
d) einen Schritt der Entnahme des Zelllysats und der Dosierung der DNA und/oder der Dosierung des Molekül der MEC und/oder eines Vorläufers des Moleküls der MEC,
e) einen Schritt der Dosierung des Moleküls der MEC, das in der extrazellulären Matrix enthalten ist, durch immunologische Technik.

12. Verwendung des Verfahrens zum Dosieren gemäß einem der vorhergehenden Ansprüche für das Screening und/oder die In-vitro-Untersuchung mindestens eines Stoffes von kosmetischem und/oder pharmazeutischem Interesse auf Grund seiner Eigenschaften der Erhöhung oder Verringerung der Menge eines Moleküls in der extrazellulären Matrix.

13. Verfahren zum Screening und/oder zur In-vitro-Untersuchung mindestens eines Stoffes von kosmetischem und/oder pharmazeutischem Interesse auf Grund seiner Eigenschaften der Erhöhung oder Verringerung der Menge eines Moleküls in der extrazellulären Matrix, umfassend mindestens die folgenden Schritte:
a) einen Schritt der Kultivierung der Zellen im Beisein des Stoffes,
b) vorzugsweise einen Schritt der Entnahme des Kulturmediums und weiter bevorzugt der Dosierung des Moleküls der MEC und/oder eines Vorläufers des Moleküls der MEC in dem so entnommenen Kulturmedium,
c) einen Schritt der Lyse der Zellen mittels einer quaternären Aminlösung,
d) einen Schritt der Entnahme des Zelllysats und der Dosierung der DNA und/oder der Dosierung des Moleküls der MEC und/oder eines Vorläufers des Moleküls der MEC,
e) einen Schritt der Dosierung des Moleküls der MEC, das in der extrazellulären Matrix enthalten ist, durch immunologische Technik,
und bei dem die Kultivierung der Zellen in dem Kulturmedium, das den kosmetischen und/oder pharmazeutischen Stoff enthält, für mindestens 24 Stunden durchgeführt wird.

14. Verwendung eines Kits zur Dosierung mittels eines immunologischen Verfahrens bei einem Verfahren zum Dosieren gemäß einem der Ansprüche 1 bis 11 oder bei einem Screening-Verfahren gemäß Anspruch 13, wobei das Kit eine quaternäre Aminlösung und einen gegen das Molekül der MEC gerichteten Antikörper, eventuell gekoppelt mit einem sekundären Antikörper, umfasst.

15. Verfahren zur Auswahl einer Lyselösung, die bei dem Verfahren zum Dosieren gemäß einem der Ansprüche 1 bis 11 oder gemäß dem Screening-Verfahren gemäß Anspruch 13 nützlich ist, **dadurch gekennzeichnet, dass** das Verfahren zum Dosieren mit einer Lyselösung eingesetzt wird, die für mindestens 10 min, vorzugsweise 10 min, zu testen ist, und das Verhältnis des Resultats der Dosierung des Moleküls der MEC, das in Schritt e) erhalten wird, zum Resultat der Dosierung der DNA in dem Zelllysat in Schritt d) mit jenem verglichen wird, das mit einer Lyselösung von Ammoniumhydroxid zu 20 mM, die für dieselbe Zeit, vorzugsweise 10 Minuten, angewandt wird, erhalten wird.

## Claims

1. A method for *in vitro* assaying at least one molecule of the extracellular matrix (ECM) synthesized by cells in culture using an immunological technique, comprising at least the steps:
a) a step of culturing the cells at confluence for at least 24 hours,
b) a preferred step of collecting the culture medium,
c) a step of cell lysis with a solution of quaternary amine,
d) a step of collecting the cell lysate and assaying DNA and/or said molecule of the ECM in the cell lysate,
e) a step of assaying said molecule of the ECM in the extracellular matrix by an immunological technique,
and wherein the cells in culture are maintained in culture at confluence for a period of time in the range from 24 to 96 hours before step c) is carried out.

2. The assay method according to claim 1, wherein step b) of collecting the culture medium is carried out.

3. The assay method according to one of the preceding claims, wherein the molecule of the ECM is selected from the proteins constituting the ECM, the glycoproteins constituting the ECM, the glycosaminoglycans constituting the ECM, the proteoglycans constituting the ECM, and the growth factors contained in the ECM, and advantageously selected from type I, III, V, VI, XII, XIV, XVI, IV and VII collagens, elastin, tropoelastin, fibrillin 1, LOX, LOXL, elastin binding protein (EBP), fibulin-3 and fibulin-5, EMILIN-1 and EMILIN-2, perlecan, versican, decorin, biglycan, lumican, fibronectin, laminin, hyaluronic acid, heparan sulfate, VEGF, PDGF, FGF-2, FGF-7, and HGF.

4. The assay method according to one of the preceding claims, wherein the cells in culture are selected from stromal cells, preferably fibroblasts, osteoblasts and/or adipoblasts, epithelial cells, preferably keratinocytes or endothelial cells, and advantageously selected from fibroblasts and/or keratinocytes and/or adipocytes.

5. The assay method according to one of claims 1 to 4, wherein an active ingredient of cosmetic and/or pharmaceutical interest is added to the culture medium, and advantageously selected from vitamin C, preferably at a dose in the range from 5 µM to 100 µM, and TGF beta, preferably at a dose in the range from 1 ng/ml to 100 ng/ml.

6. The assay method according to one of claims 2 to 5, wherein the molecule of the ECM and/or a precursor of the molecule of the ECM is assayed by an immunological method in the culture medium collected in step b), preferably by an ELISA-type assay.

7. The assay method according to one of the preceding claims, wherein the solution of quaternary amine is a solution of ammonium, optionally in the form of salts, and advantageously selected from an ammonium chloride solution, an ammonium hydroxide solution, and mixtures thereof.

8. The assay method according to one of the preceding claims, wherein the solution of quaternary amine is at a concentration in the range from 10 mM to 200 mM.

9. The assay method according to one of the preceding claims, wherein the solution of quaternary amine is applied to the cells for a period of time in the range from 5 to 60 minutes.

10. The assay method according to one of the preceding claims, wherein step e) of assay of said molecule contained in the extracellular matrix using an immunological technique is carried out by the time-resolved fluorescence (TRF) method.

11. The assay method according to one of the preceding claims, comprising at least the steps:
a) a step of culturing the cells, and preferably culturing the cells at confluence for at least 24 hours,
b) a step of collecting the culture medium and of assaying the molecule of the ECM and/or a precursor of the molecule of the ECM,
c) a step of cell lysis with a solution of quaternary amine,
d) a step of collecting the cell lysate and of assaying the DNA and/or of assaying the molecule of the ECM and/or a precursor of the molecule of the ECM,
e) a step of assaying said molecule of the ECM contained in the extracellular matrix by an immunological technique.

12. Use of the assay method according to one of the preceding claims for screening and/or studying *in vitro* at least one ingredient of cosmetic and/or pharmaceutical interest for its properties of increasing or decreasing the quantity of a molecule in the extracellular matrix.

13. A method of screening and/or studying *in vitro* at least one ingredient of cosmetic and/or pharmaceutical interest for its properties of increasing or decreasing the quantity of a molecule in the extracellular matrix, comprising at least the steps:
a) a step of culturing the cells in the presence of the ingredient,
b) preferably a step of collecting the culture medium and more preferably of assaying the molecule of the ECM and/or a precursor of the molecule of the ECM in said culture medium thus collected,
c) a step of cell lysis with a solution of quaternary amine,
d) a step of collecting the cell lysate and of assaying the DNA and/or of assaying the molecule of the ECM and/or a precursor of the molecule of the ECM,
e) a step of assaying said molecule of the ECM contained in the extracellular matrix by an immunological technique,
and wherein the cell culture in the culture medium containing the cosmetic and/or pharmaceutical ingredient is carried out for at least 24 hours.

14. Use of a kit for assay by an immunological method in the assay method according to one of claims 1 to 11 or in a screening method according to claim 13, said kit comprising a solution of quaternary amine and an antibody directed against the molecule of the ECM, optionally coupled to a secondary antibody.

15. A method for selecting a lysis solution useful in the assay method according to one of claims 1 to 11 or according to the screening method according to claim 13, **characterized in that** the assay method is carried out with a lysis solution to be tested for at least 10 min, preferably 10 min, and the ratio of the result of the assay of the molecule of the ECM obtained in step e) to the result of the assay of the DNA in the cell lysate in step d) is compared with that obtained with an ammonium hydroxide lysis solution at 20 mM applied for the same period of time, preferably 10 minutes.
